(19) Europäisches Patentamt
European Patent Office
Office européen des brevets

(11) **EP 1 352 635 A2**

(12) **DEMANDE DE BREVET EUROPEEN**

(43) Date de publication:
**15.10.2003 Bulletin 2003/42**

(51) Int Cl.[7]: **A61K 7/13**

(21) Numéro de dépôt: **03290873.3**

(22) Date de dépôt: **08.04.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **09.04.2002 FR 0204427**

(71) Demandeur: **L'OREAL**
**75008 Paris (FR)**

(72) Inventeurs:
- **Dreher, Frank**
**75005 Paris (FR)**
- **Pruche, Francis**
**60300 Senlis (FR)**

(74) Mandataire: **Michelet, Alain et al**
**Cabinet Harlé et Phélip**
**7, rue de Madrid**
**75008 Paris (FR)**

(54) **Composition de coloration des fibres kératiniques comprenant un système limitant le passage transcutané d'un colorant d'oxydation**

(57) La composition comprend, dans un milieu physiologiquement acceptable, une quantité efficace d'au moins un colorant d'oxydation orthodiphénol choisi parmi les composés comportant au moins un cycle aromatique ayant au moins deux groupes hydroxyles portés par deux atomes de carbone consécutifs du cycle aromatique et, en tant qu'agent de réduction de l'absorption par la peau et du passage transcutané du colorant d'oxydation d'une quantité efficace d'un système comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec} [Mn(II)] \text{ et } [Zn(II)] \neq 0.$$

Application à la coloration capillaire.

EP 1 352 635 A2

## Description

La présente invention concerne d'une manière générale l'utilisation dans une composition pour la coloration des fibres kératiniques comprenant au moins un colorant d'oxydation orthodiphénol, en tant qu'agent de limitation du passage transcutané du colorant d'oxydation, d'un système comprenant au moins un sel ou oxyde de Mn(II) ou Zn(II), et au moins un hydrogénocarbonate alcalin ou alcalino-terreux, pour obtenir une meilleure tolérance du colorant et éviter un tachage de la peau.

Certains colorants d'oxydation sont susceptibles de provoquer des réactions d'inconfort, d'irritation, de sensibilisation ou de tachage du cuir chevelu, même si toutes les précautions sont prises pendant une coloration pour éviter le contact avec la peau, que leur possibilité d'être en contact avec la peau est très courte, et qu'ils sont rincés juste après coloration. De telles réactions d'intolérance sont susceptibles d'apparaître lorsque le colorant atteint la barrière cutanée.

Pour des colorants d'oxydation susceptibles de provoquer de telles réactions, la pénétration dans la peau de ces colorants, bien que réduite, peut être encore limitée, ce qui aurait pour conséquence une meilleure tolérance du colorant et permettrait d'éviter un tachage de la peau.

On connaît de nombreuses familles de composés qui ont la propriété d'activer la pénétration dans la peau ou les fibres kératiniques d'un agent actif contenu dans une composition cosmétique et/ou pharmaceutique.

Par contre, on connaît très peu de familles de composés possédant l'activité inverse, c'est-à-dire réduisant effectivement la pénétration d'un agent actif d'une composition cosmétique et/ou pharmaceutique dans la peau et/ou les fibres kératiniques.

Même si de nombreux progrès ont été effectués dans le domaine de la coloration des fibres kératiniques (comme par exemple les cheveux), et que les colorants d'oxydation actuellement disponibles dans le commerce sont très fiables, il serait toutefois souhaitable de disposer d'un agent qui, utilisé avec une composition cosmétique de coloration des fibres kératiniques, aurait des propriétés de réduction de la pénétration dans la peau du colorant d'oxydation contenu dans la composition de coloration, et qui permettrait ainsi d'éliminer les effets secondaires d'intolérance et de tachage de la peau.

La présente invention vise justement à la satisfaction d'un tel besoin.

Or, la demanderesse a trouvé de manière surprenante qu'un système comprenant au moins un sel et/ou oxyde de manganèse (II) et /ou de zinc (II) et un hydrogénocarbonate alcalin et/ou alcalino-terreux présente des propriétés de réduction de la pénétration dans la peau des colorants d'oxydation lorsque ce système est ajouté à des compositions de coloration des fibres kératiniques contenant ces colorants d'oxydation, sans nuire à la coloration des fibres kératiniques.

La composition pour la coloration des fibres kératiniques selon l'invention comprend, dans un milieu physiologiquement acceptable, une quantité effective d'au moins un colorant d'oxydation orthodiphénol et une quantité effective d'un système limitant le passage transcutané du colorant d'oxydation comprenant un premier constituant choisi parmi les sels et oxydes de Mn(II) et/ou Zn(II) et un second constituant choisi parmi les hydrogénocarbonates alcalins, les hydrogénocarbonates alcalino-terreux et leurs mélanges, les proportions du premier constituant et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

Généralement, le rapport $\frac{[Mn(II)]}{[HCO_3]}$ varie de $10^{-5}$ à $10^{-1}$, de préférence de $10^{-3}$ à $10^{-2}$ et est typiquement de l'ordre de $5.10^{-3}$.

Dans le cas de Zn(II), le rapport $\frac{[Zn(II)]}{[HCO_3]}$ est en général d'un ordre de 10 à 100 fois supérieur au rapport dans le cas de Mn(II).

Typiquement, ce rapport est de $10^{-4}$ ou plus, de préférence $10^{-3}$ ou plus, et de préférence de l'ordre de $5.10^{-1}$.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Généralement, la concentration molaire en Mn(II), Zn(II), ou Mn(II) + Zn(II) dans la composition finale varie de $10^{-3}$ à 10 mM/l, de préférence de $10^{-2}$ à 1 mM/l.

Lorsqu'on utilise seulement un ou plusieurs sels ou oxydes de Mn(II), la concentration molaire en Mn(II) dans la composition finale peut être choisie dans un domaine de $10^{-3}$ à $10^{-1}$ mM/l, de préférence $10^{-2}$ à $10^{-1}$ mM/l.

De préférence, lorsqu'on utilise uniquement un ou plusieurs sels ou oxydes de Zn(II), la concentration en Zn (II) dans la composition finale est de $5.10^{-2}$ à 10 mM/l, mieux de $5.10^{-1}$ à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétrahydraté, le lactate de Mn(II) trihydraté, le phosphate de Mn (II), l'iodure de Mn(II), le nitrate de Mn(II) trihydraté, le bromure de Mn(II) et le perchlorate de Mn(II) tétrahydraté, et le sulfate de Mn(II) monohydraté.

Les sels particulièrement préférés sont $MnCl_2$ et $ZnCl_2$ et tout particulièrement $MnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na.

De manière générale, les colorants d'oxydation des compositions de l'invention sont tous des composés ou mélanges de composés qui, en présence d'oxygène, par exemple de l'oxygène de l'air, s'oxydent pour donner un composé ou un mélange de composés colorés.

Les colorants d'oxydation orthodiphénols sont des composés comportant au moins un cycle aromatique dont au moins deux carbones consécutifs portent un groupe hydroxyle. De préférence, le cycle aromatique est un cycle benzénique ou un cycle aromatique condensé.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Les colorants d'oxydation orthodiphénols préférés peuvent être représentés par la formule :

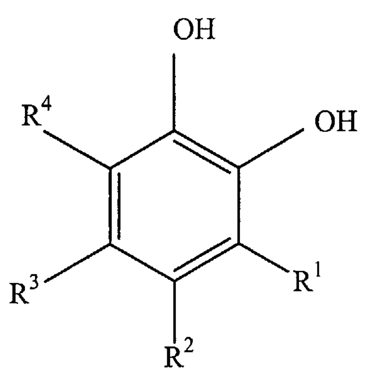

(I)

dans laquelle les substituants $R^1$ à $R^4$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant un ou plusieurs atomes de silicium, où deux des substituants $R^1$ à $R^4$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_2$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_2$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphenylsiloxane, polydiméthylphénylsiloxane, stéaroxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocyliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les colorants orthodiphénols préférés sont

- les flavanols comme la catéchine et le gallate d'épichatéchine,
- les flavonols comme la quercétine,
- les anthocyanidines comme la péonidine,
- les anthocyanines, par exemple l'oenine,
- les hydroxybenzoates, par exemple l'acide gallique,
- les flavones comme la lutéoline,
- les iridoïdes comme l'oleuropéine,

ces produits pouvant être osylés (par exemple glucosylés) et /ou sous forme d'oligomères (procyanidines) ;

- les hydroxystilbènes, par exemple le tétrahydroxy-3,3',4,5'-stilbène, éventuellement osylés (par exemple glucosylés) ;
- la 3,4-dihydroxyphénylalanine et ses dérivés ;
- la 2,3-dihydroxyphénylalanine et ses dérivés ;
- la 4,5-dihydroxyphénylalanine et ses dérivés ;
- le 4,5-dihydroxyindole et ses dérivés ;
- le 5,6-dihydroxyindole et ses dérivés ;
- le 6,7-dihydroxyindole et ses dérivés ;
- le 2,3-dihydroxyindole et ses dérivés ;
- les dihydroxycinnnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ;
- les hydroxyxanthones ; et
- les mélanges de ceux-ci.

Lorsque les colorants présentent des formes D et L, les deux formes peuvent être utilisées dans les compositions selon l'invention.

Les colorants d'oxydation orthodiphénols particulièrement préférés sont le 5,6-dihydroxyindole et l'acide 5,6-dihydroxyindole carboxylique.

Les polymères formés en particulier avec la catéchine, l'acide gallique et leurs dérivés (tannins) ont des propriétés antimicrobiennes par emprisonnement des microorganismes lors de la polymérisation.

Les colorants d'oxydation orthodiphénols peuvent être contenus dans des extraits de plantes, fruits, agrumes,

légumes ou des mélanges de ces extraits.

Parmi les extraits de plantes, on peut citer les extraits de rose et de thé.

Parmi les extraits de fruits, on peut citer les extraits de pomme, de raisin (en particulier de pépins de raisin) et de banane.

Parmi les extraits de légumes, on peut citer l'extrait de pomme de terre.

On peut également utiliser des mélanges d'extraits de plantes et/ou de fruits tels que des mélanges d'extraits de pomme et de thé et des mélanges d'extraits de raisin et de pomme.

Suivant les parties de fruits utilisés, par exemple pulpe ou pépins de raisin, la coloration obtenue est différente.

Les compositions conformes à l'invention peuvent également comprendre, outre le colorant d'oxydation orthodiphénol, au moins un second colorant d'oxydation autre que le colorant orthodiphénol choisi parmi les bases d'oxydation, de type para ou ortho, les coupleurs et leurs mélanges.

Les bases d'oxydation sont des composés qui ne sont pas des colorants en eux-mêmes, mais qui forment un colorant par un processus de condensation oxydative, soit sur eux-mêmes, soit en présence d'un coupleur ou modificateur.

Les bases d'oxydation sont des composés qui comportent des groupements fonctionnels, soit deux groupements amino, soit un groupement amino et un groupement hydroxy en position para ou ortho l'un par rapport à l'autre.

La nature de ces bases d'oxydation n'est pas critique. Elles peuvent notamment être choisies parmi les ortho- et para-phénylènediamines, les bases doubles, les ortho- et para-aminophénols, les bases hétérocycliques ainsi que les sels d'addition de tous ces composés avec un acide.

A titre de paraphénylènediamines, on peut notamment citer les paraphénylènediamines de formule (II) suivante et leurs sels d'addition avec un acide :

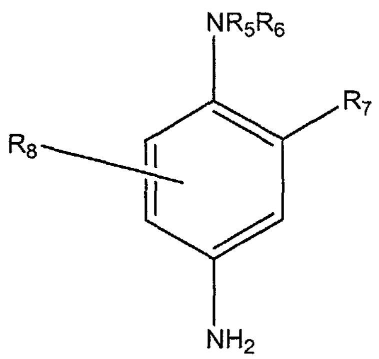

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;,
- $R_8$ représente un atome d'hydrogène, d'halogène ou un radical alkyle en $C_1$-$C_4$ ;
- $R_5$ et $R_6$ peuvent également former avec l'atome d'azote qui les porte un hétérocycle azoté à 5 ou 6 chaînons éventuellement substitué par un ou plusieurs groupements alkyle, hydroxy ou uréido.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (II) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthylparaphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β -hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-(β-hydroxyéthyl)-paraphénylènediamine, la 2-chloroparaphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide :

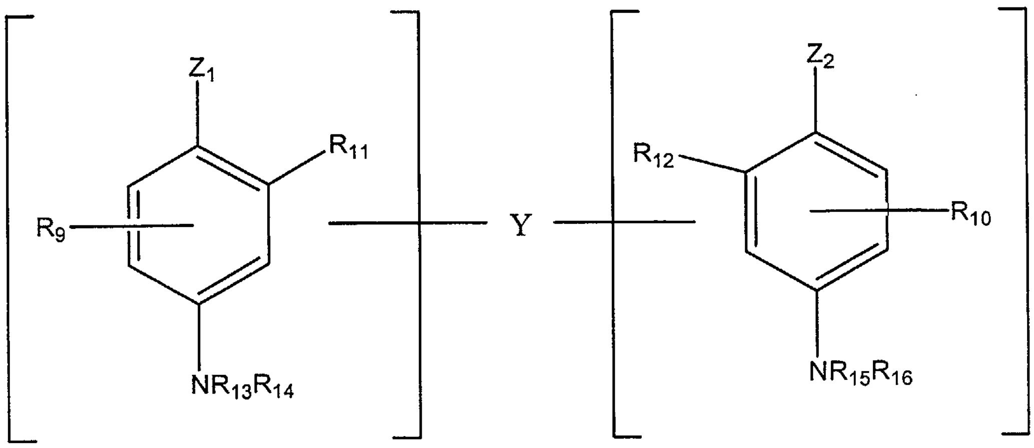

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ; étant entendu que les composés de formule (III) ne comportent qu'un seul bras de liaison Y par molécule.

Parmi les groupements azotés de la formule (III) ci-dessus, on peut citer notamment les radicaux amino, monoalkyl($C_1$-$C_4$)amino, dialkyl($C_1$-$C_4$)amino, trialkyl($C_1$-$C_4$)amine, monohydroxyalkyl($C_1$-$C_4$)amine, imidazolinium et ammonium.

Parmi les bases doubles de formules (III) ci-dessus, on peut plus particulièrement citer le N,N'-bls-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols, on peut notamment citer les para-aminophénols répondant à la formule (IV) suivante, et leurs sels d'addition avec un acide :

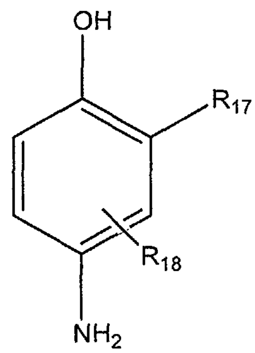

(IV)

dans laquelle :

- $R_{17}$ représente un atome d'hydrogène,un atome d'halogène tel que le fluor ou le chlore, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$, et
- $R_{18}$ représente un atome d'hydrogène ou un atome d'halogène tel que le fluor

ou le chlore, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthylbenzène, le 5-acétamido-2-amino-phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1026978 et GB-1153196, comme la 2,5-diamino-pyridine, la 2-(4-méthoxyphényl)amino-3-aminopyridine, la 2,3-diamino-6-méthoxy-pyridine, la 2-(a-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE-2359399 ou japonais JP 88-169571 et JP-9110659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5,N7,N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE-3843892, DE-4133957 et demandes de brevet WO 94/08969, WO 94/08970, FR-A-2 733 749 et DE-19543988 comme

le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans les compositions conformes à l'invention peuvent être choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide, ces composés étant différents des composés orthodihydroxylés de l'invention.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale prête à l'emploi et encore plus préférentiellement de 0,001 à 10% environ.

Les bases d'oxydation et les coupleurs constituent des colorants d'oxydation. Les sels d'addition avec un acide de ces colorants d'oxydation sont notamment choisis parmi les chlorhydrates, les bromhydrates, les sulfates et les tartrates, les lactates et les acétates.

Les compositions conformes à l'invention peuvent en outre renfermer un ou plusieurs colorants directs notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Les compositions selon l'invention peuvent également contenir une quantité effective d'au moins un acide aminé en particulier comportant au moins un groupe thiol (SH) et de préférence un seul groupe thiol, ces acides aminés pouvant être sous la forme de chlorhydrates et/ou au moins une protéine, en particulier un peptide.

Les acides aminés préférés selon l'invention sont les acides aminés qui contiennent une fonction amine en position α par rapport à une fonction acide carboxylique.

Les acides aminés préférés peuvent être représentés par la formule

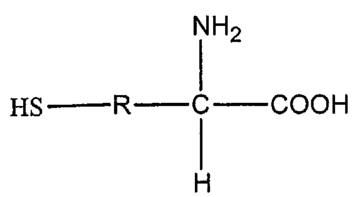

(V)

dans laquelle -R est un radical hydrocarboné divalent, linéaire ou ramifié, par exemple en $C_1$-$C_{10}$, de préférence en $C_1$-$C_6$, tel qu'un radical méthylène, éthylène, butylène, éthylidène, propylidène, un radical cyclique saturé divalent, éventuellement substitué, par exemple en $C_4$-$C_8$, un groupe aromatique divalent, éventuellement substitué, tel qu'un radical phénylène, tolylène, xylylène.

Parmi les acides aminés préférés pour les compositions de l'invention, on peut citer la cystéine et ses dérivés, en particulier la L-cystéine et le chlorhydrate de L-cystéine.

Parmi les protéines, on peut citer le gluthation et ses dérivés et la protéine de soja.

Les proportions relatives d'acide aminé et/ou de protéine et de colorant d'oxydation dans les compositions de l'invention peuvent varier dans de larges mesures en fonction de la coloration voulue. Généralement, le rapport molaire acide aminé/colorant d'oxydation variera de 0,001 à 50, de préférence de 0,01 à 5, et mieux de 0,05 à 2,5.

En général, la teneur en acide aminé à groupe thiol dans la composition finale est d'au moins 0,01 micromole par millilitre, de préférence au moins 0,1 micromole/ml.

En faisant varier la nature des précurseurs de colorant et des acides aminés de la composition et la proportion relative d'acide aminé et de précurseur de colorant, on peut obtenir toute une palette de teintes.

Les compositions selon l'invention peuvent en outre comprendre une ou plusieurs enzymes.

L'enzyme ou les enzymes présentes dans les compositions selon l'invention peuvent être toutes enzymes présentant une activité propigmentation.

L'activité propigmentation peut se définir comme l'activité enzymatique qui catalyse l'oxydation d'un substrat pour conduire à la formation de pigments.

Les enzymes peuvent notamment être choisis parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases, les superoxydesdimutases et leurs mélanges, ou parmi des extraits végétaux ou animaux contenant des enzymes précitées, en présence éventuelle d'un donneur (ou substrat) nécessaire au fonctionnement desdites enzymes tel que par exemple la L-tyrosine ou la L-DOPA.

Les enzymes utilisées selon l'invention peuvent être d'origine animale, microbiologique (bactérienne, fongique ou virale) ou synthétique (obtenue par synthèse chimique ou biotechnologique).

La ou les enzymes peuvent être utilisées sous forme cristalline pure ou sous une forme diluée dans un diluant inerte pour ladite enzyme.

A titre d'exemple d'uricases, on peut notamment citer l'uricase extraite de foie de sanglier, l'uricase d'Arthrobacter globiformis, ainsi que l'uricase d'Aspergillus flavus.

A titre d'exemple de sources de choline oxydase, on peut notamment citer le foie de rat, les bactéries telles que Arthrobacter globiformis, Achromobacter cholinophagum ou Alcaligenes, et les champignons tels que Cylindrocarpon didynum.

A titre d'exemple de sources de sarcosine oxydase, on peut notamment citer les bactéries telles que Arthrobacter et en particulier Arthrobacter ureafaciens et Arthrobacter globiformis, Streptomyces, Bacillus, Pseudomonas, Corynebacterium ou Alcaligenes tels que par exemple Alcaligenes denitrificans, et les champignons tels que Cylindrocarpon didynum.

A titre d'exemple de sources de bilirubine oxydase, on peut notamment citer la muqueuse intestinale et le foie de rat, les bactéries telles que Myrothecium verucania, Myrothecium cinctum, et Myrothecium roridum.

Parmi les laccases d'origine végétale utilisables selon l'invention, on peut citer les laccases produites par des végétaux effectuant la synthèse chlorophylienne telles que celles indiquées dans la demande de brevet FR-A-2.694.018.

On peut notamment citer les laccases extraites d'Anacardiacées, de Podocarpacées, de Rosmarinus off., de Solanum tuberosum, dIris sp., de Coffea sp., de Daucus carrota, de Vinca minor, de Persea americana, de Catharenthus roseus, de Musa sp., de Malus pumila, de Gingko biloba, et de Monotropa hypopithys (sucepin).

Parmi les laccases d'origine microbienne (notamment fongique), ou obtenues par biotechnologie utilisables selon l'invention, on peut citer les laccases de Polyporus versicolor, de Rhizoctonia praticola et de rhus vernicifera telles que décrites par exemple dans les demande de brevet FR-A-2.112.549 et EP-A-504.005 ; les lacases décrites dans les demandes de brevet WO95/07988, WO95/33836, WO95/33837, WO96/00290, WO97/19998 et WO97/19999, dont le contenu fait partie intégrante de la présente description comme par exemple les laccases de Scytalidium, de Polyporus pinsitus, de Myceliophthora thermophila, de Rhizoctonia solani, de Pyricularia orizae, et leurs variantes.

On choisira plus préférentiellement les laccases d'origine microbienne ou celles obtenues par biotechnologie.

Dans une forme de réalisation particulièrement préférée de l'invention, l'enzyme utilisée correspond à la tyrosinase (nomenclature EC 1.14.18.1). Par tyrosinase, il faut entendre dans la présente invention toute enzyme présentant une activité tyrosinase, cette enzyme pouvant présenter d'autres activités enzymatiques. L'activité tyrisonase peut se définir comme l'activité enzymatique qui catalyse l'oxydation de la tyrosine pour conduire à la formation du précurseur de mélanine : la Dopaquinone.

A titre d'exemple de sources de tyrosinase, on peut notamment citer la pomme de terre, les champignons, les micro-organismes tels que Neurospora crassa, etc.

La quantité d'enzyme présente dans la composition finale peut varier largement mais est généralement de $5.10^{-3}$ à 5 mg, de préférence $5.10^{-2}$ à 0,5 mg par millilitre de composition finale.

Les compositions tinctoriales peuvent en outre plus particulièrement contenir, au moins un agent tensio-actif dans la proportion d'au moins 0,01% en poids et de préférence un agent tensio-actif de nature non-ionique.

Les agents tensio-actifs peuvent être choisis parmi les agents tensio-actifs anioniques, cationiques, non-ioniques, amphotères ou leurs mélanges, et de préférence parmi les tensio-actifs non ioniques.

Parmi ces agents tensio-actifs, on peut citer les alkylbenzènesulfonates, les alkylnaphtalènesulfonates, les sulfates, les éthers sulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les quantités d'agents tensio-actifs présents dans la composition selon l'invention peuvent varier de 0,01 à 40% et de préférence de 0,5 à 30% du poids total de la composition.

Les agents épaississants que l'on peut ajouter dans les compositions conformes à l'invention, peuvent être choisis parmi l'alginate de sodium, la gomme arabique, les dérivés de cellulose, les polymères d'acide acrylique, la gomme de xanthane. On peut également utiliser les agents épaississants minéraux tels que la bentonite.

Ces agents épaississants sont présents de préférence dans des proportions comprises entre 0,1 et 5% et en particulier entre 0,2 et 3% en poids par rapport au poids total de la composition.

Ces compositions peuvent également contenir d'autres adjuvants cosmétiquement acceptables, tels que par exemple des agents de pénétration, des parfums, des tampons, etc.

Les compositions selon l'invention peuvent également comprendre tout adjuvant classique, en proportion usuelle, qui ne nuit pas aux propriétés recherchées, en particulier à l'effet colorant des compositions.

La composition colorante peut encore contenir une quantité efficace d'autres agents, par ailleurs antérieurement connus en coloration d'oxydation, tels que divers adjuvants usuels comme des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ces filtres UV organiques peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4,367,390, EP-0.863.145, EP-0.517.104, EP-0.570.838, EP-0.796.851, EP-0.775.698, EP-0.878.469, EP-0.933.376 et EP-0.893.119 ; les dérivés de la benzophénone ; les dérivés de β ,β'-diphénylacrylate, les dérivés de benzimidazole ; les dérivés bis-benzoazolyle tels que ceux décrits dans les brevets EP-0.669.323 et US 2,463,264 ; les dérivés de méthylène bis-(hydroxyphénylbenzotriazole) tels que ceux décrits dans les demandes US 5,237,071, US 5,166,355, GB-2303549, DE-19726184 et EP-0.893.119 ; les dérivés de l'acide p-aminobenzoïque ; les dimères dérivés d'α-alkylstyrène tels que décrits dans la demande de brevet DE-19855649 ; les polymères hydrocarbonés filtres et les silicones filtres tels que ceux décrits notamment dans la demande WO 93/04665. On peut aussi utiliser des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-0.518.772 et EP-0.518.773.

Bien entendu, l'homme de l'art veillera à choisir le ou les éventuels composés complémentaires mentionnés précédemment, de manière telle que les propriétés avantageuses attachées intrinsèquement à la composition tinctoriale selon l'invention ne soient pas, ou substantiellement pas, altérées par la ou les adjonctions envisagées.

La quantité de colorants d'oxydation, en particulier orthodiphénol, dans la composition finale doit être suffisante pour obtenir une coloration visible. Cette quantité peut varier dans de larges mesures en fonction de la nature du colorant d'oxydation et de l'intensité voulue pour la coloration.

En général, on obtiendra une coloration convenable lorsque la quantité de colorant est telle que la teneur en colorant d'oxydation orthodiphénol dans la composition de coloration finale est d'au moins 0,1 micromole, de préférence d'au moins 1 micromole par millilitre de composition finale.

Typiquement, la quantité de colorant d'oxydation orthodiphénol dans la composition finale varie de 1 mM à 10 mM par litre et généralement de l'ordre de 5 mM par litre.

En faisant varier la nature des différents colorants d'oxydation et leurs proportions dans la composition, on peut faire varier la couleur de la composition de coloration finale. On obtient ainsi une palette de couleurs.

Par exemple, avec un ratio 1/10 d'acide chlorogénique et de catéchine, on obtient une coloration marron claire et avec un ratio 1/1 une coloration acajou.

Le milieu physiologiquement acceptable est un milieu solide ou liquide ne nuisant pas à la propriété de coloration des colorants d'oxydation ni à l'effet réducteur de l'absorption et du passage transcutané du système.

Le milieu physiologiquement acceptable est de préférence un milieu solubilisant du colorant d'oxydation et à propriété bactériostatique.

Parmi les solvants des colorants convenant pour la formulation des compositions selon l'invention, on peut citer l'eau, les alcools, les solvants et leurs mélanges.

Les alcools sont de préférence des alcanols inférieurs ($C_1$-$C_6$) tels que l'éthanol et l'isopropanol et les alcanediols tels que l'éthylèneglycol, le propylène glycol et le pentane diol.

Parmi les solvants, on peut citer les éthers, les esters (en particulier les acétates), le diméthylsulfoxyde (DMSO), la N-méthylpyrrolidone (NMP), les cétones (en particulier l'acétone) et leurs mélanges.

Le milieu physiologiquement acceptable comprend de préférence de l'eau (en particulier distillée ou permutée) ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20% en poids.

Le milieu physiologiquement acceptable peut être un milieu solide tel qu'un excipient pour la formulation de galets et comprimés, en particulier effervescents.

De préférence, les compositions selon l'invention sont exemptes d'agents de chélation des sels de Mn(II) et/ou Zn(II) utilisés, car ces agents tendent à inhiber l'oxydation des colorants.

Pour révéler la coloration des compositions suivant l'invention, il suffit de mettre la composition contenant au moins un colorant d'oxydation et une quantité efficace du système selon l'invention, en présence d'un milieu oxydant tel qu'un milieu contenant de l'oxygène (par exemple l'oxygène de l'air).

Les compositions selon l'invention sont utiles pour la coloration des fibres kératiniques tels que les cheveux, les cils, les sourcils et les poils.

Pour la coloration des fibres kératiniques, différents procédés d'application des compositions selon l'invention peuvent être utilisés.

Selon un premier procédé, on applique sur les fibres kératiniques, en présence d'oxygène par exemple l'oxygène de l'air, une composition comprenant tous les ingrédients de la composition, c'est-à-dire à la fois le ou les colorants d'oxydation et le système limitant le passage transcutané.

Selon un second procédé, on peut en premier lieu appliquer sur les fibres kératiniques et/ou sur le cuir chevelu un film du système limitant le passage transcutané dans un milieu physiologiquement acceptable, puis sur les fibres kératiniques, un film d'une composition de base d'un ou plusieurs colorants d'oxydation dans un milieu physiologiquement acceptable qui, en présence d'oxygène, révélera la coloration de la composition de base.

On peut, mais cela n'est pas recommandé, inverser l'ordre d'application des films.

L'application des films peut se faire par tout moyen connu, en particulier par pulvérisation.

Les compositions selon l'invention peuvent se présenter et être conditionnées sous différentes formes, telles que crèmes, masques, aérosols, lotions etc....

En particulier, les compositions selon l'invention peuvent se présenter sous la forme de deux composants séparés, un premier composant comprenant le système dissous dans un milieu physiologiquement acceptable et un deuxième composant comprenant le colorant d'oxydation dissous dans un milieu physiologiquement acceptable.

Selon une première réalisation, les compositions selon l'invention peuvent être conditionnées sous forme d'aérosol à un seul compartiment dans lequel se trouvent la composition renfermant le ou les colorants et le système réduisant l'absorption et le passage transcutané des colorants, et un gaz propulseur inerte classique tel que de l'azote, un hydrocarbure saturé comme l'isopropane ou un hydrocarbure fluoré, par exemple un Fréon®.

Dans une seconde réalisation, la composition selon l'invention peut être conditionnée sous forme d'un kit comportant deux conteneurs distincts, l'un pour la composition de base contenant le ou les colorants, l'autre pour le système, la composition de base et le système étant mélangés ou appliqués successivement au moment de l'emploi.

Dans une troisième réalisation, la composition peut être contenue dans un système à pompe à un seul compartiment, sans reprise d'air, ou dans un système à pompe à deux compartiments, la composition de base étant dans un compartiment et le système réduisant l'absorption et le passage transcutané dans l'autre.

Dans une quatrième réalisation, la composition selon l'invention peut se présenter sous forme de galets. Chaque galet peut comporter, mélangé à un excipient, le ou les colorants et le système, l'excipient empêchant la réaction en présence d'oxygène ou le ou les colorants et le système sont contenus dans des galets distincts.

En délitant soit le galet unique soit un galet de chacun des constituants par exemple dans de l'eau, on réalise la composition colorante selon l'invention.

Les galets, comme cela est classique, peuvent être des galets effervescents.

L'excipient utilisé peut être tout excipient classique tel qu'un mélange de talc, de stéarate (en particulier de magnésium), d'acide citrique et/ou tartrique, et d'hydrogèno carbonate alcalin et / ou alcalino terreux.

La quantité d'acide citrique et/ou tartrique présente doit être telle qu'il n'y ait pas une neutralisation de l'hydrogénocarbonate résultant en un manque d'hydrogénocarbonate libre par rapport au Mn(II) et/ou Zn(II), c'est à dire que l'acide citrique et/ou tartrique est en quantité sous stoedriométrique par rapport à l'hydrogénocarbonate alcalin et/ou alcalino-teneux.

D'autre part, comme l'eau, en particulier l'eau du robinet et certaines eaux de source ou minérales, contiennent généralement du manganèse (II), il est parfois suffisant de mettre dans l'eau le seul galet contenant de l'hydrogénocarbonate et le ou les colorants d'oxydation, la teneur en Mn(II) du système réduisant l'absorption et le passage transcutané étant alors fournie par le Mn(II) présent dans l'eau.

De même, certains extraits végétaux (par exemple des extraits de feuilles de thé) peuvent contenir des quantités importantes de manganèse (II). En fonction de ces teneurs, un ajustement des concentrations du système est effectué de manière à avoir un résultat satisfaisant.

Bien évidemment, on peut faire varier l'intensité de la coloration en délitant plusieurs galets dans l'eau.

D'autre part, la vitesse de coloration peut être accélérée en ajoutant à la composition un composé ou une formulation de composés engendrant de l'oxygène, par exemple par contact avec de l'eau. Ainsi, on peut incorporer un tel composé ou formulation, par exemple du peroxyde de sodium, dans un galet.

La suite de la description se réfère à la figure unique qui est un graphe du taux de pénétration d'une composition de coloration selon l'invention et d'une composition analogue ne comportant pas le système de limitation du passage transcutané du colorant d'oxydation.

**EXEMPLE**

i) Réalisation de l'expérience

Le mélange de 5,6-dihydroxyindole avec le système de limitation du passage transcutané a été préparé frais par ajout d'un volume équivalent de système (= 1 mM $MnCl_2$ dans 1M $NaHCO_3$ ; pH = 10) à une solution aqueuse de 10 mM de 5,6-dihydroxyindole. Puis, il a été appliqué sur de la peau reconstruite (par exemples Epiderm® de Mattek, Inc., Episkin® d'Episkin SNC ou Skinethic® de Skinethic Laboratories) montée dans des cellules de diffusion en mode statique à une dose de 250 mg $cm^{-2}$ pendant 16 heures. L'analyse des taux de pénétration de 5,6-dihydroxyindole à travers la peau a été effectuée par spectrophotométrie VIS au travers de ses produits d'oxydation. La gamme de 5,6-dihydroxyindole a été préparée comme suit. Une série de dilutions de 0 à 0.5 mM 5,6-dihydroxyindole dans du liquide récepteur a été laissée pendant 16 heures à 32°C pour formation de produits d'oxydation en forme de pigment. Ces pigments absorbent la lumière visible (VIS) (effet « scattering ») en fonction de leur concentration, qui est directement reliée à la concentration initiale de 5,6-dihydroxyindole. Ainsi, la gamme de 5,6-dihydroxyindole a été obtenue à travers l'absorption VIS de ces pigments formés après oxydation de 5,6-dihydroxyindole. Le mélange 1 : 1 entre 10 mM 5,6-dihydroxyindole et d'eau distillée a servi comme contrôle.

ii) Résultats

Les résultats des mélanges contenant du 5,6-dihydroxyindole à une concentration finale de 5 mM sont montrés dans la Figure 1. A 5 mM de 5,6-dihydroxyindole de concentration finale, le taux de pénétration du 5,6-dihydroxyindole est d'environ 3 % de la dose appliquée sans système (= mélange de contrôle) et inférieure à 0,5% avec le système après une application de 16 heures à une dose infinie. La pénétration du 5,6-dihydroxyindole à travers la peau a été donc significativement réduite par la présence du système.

**Revendications**

1. Utilisation dans une composition pour la coloration des fibres kératiniques, comprenant dans un milieu physiologiquement acceptable une quantité effective d'au moins un colorant d'oxydation orthodiphénol, en tant qu'agent limitant le passage transcutané du colorant d'oxydation, d'une quantité effective d'un système comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituant étant telles que :

$$\frac{[Mn(II)]}{[HCO_3]} \leq 1 \text{ avec } [Mn(II)] \neq 0$$

$$\frac{[Zn(II)]}{[HCO_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition,
la limitation du passage transcutané du colorant d'oxydation permettant d'obtenir une meilleure tolérance du colorant d'oxydation et d'éviter un tâchage de la peau.

**2.** Utilisation selon la revendication 1, **caractérisée en ce que** le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

**3.** Utilisation selon la revendication 1 ou 2, **caractérisée en ce que** le rapport $\frac{[Zn(II)]}{[HCO_3]}$ varie de $10^{-4}$ à < 1, de préférence de $10^{-3}$ à < 1, et mieux est de l'ordre de $5.10^{-1}$.

**4.** Utilisation selon l'une quelconque des revendications précédentes,
**caractérisée en ce que** le rapport $\frac{[Mn(II)+Zn(II)]}{[HCO_3]}$ varie de $10^{-5}$ à $10^{-1}$, de préférence $10^{-3}$ à $10^{-2}$.

**5.** Utilisation selon l'une quelconque des revendications précédentes, **caractérisée en ce que** les sels de Mn(II) et de Zn(II) sont choisis parmi les chlorure, fluorure, iodure, sulfate, phosphate, nitrate, perchlorate, les sels d'acides carboxyliques et leurs mélanges.

**6.** Utilisation selon la revendication 5, **caractérisée en ce que** le sel de Mn(II) et/ou de Zn(II) est le chlorure.

**7.** Utilisation selon la revendication 5, **caractérisée en ce que** les sels d'acides carboxyliques sont des sels d'acides carboxyliques hydroxylés.

**8.** Utilisation selon la revendication 7, **caractérisée en ce que** le sel d'acide carboxylique hydroxylé est le gluconate.

**9.** Utilisation selon l'une quelconque des revendications précédentes, **caractérisée en ce que** l'hydrogénocarbonate est choisi parmi l'hydrogénocarbonate de sodium, l'hydrogénocarbonate de potassium et leurs mélanges.

**10.** Utilisation selon l'une quelconque des revendications précédentes, **caractérisée en ce que** le colorant orthodiphénol comporte un cycle benzénique ou un cycle aromatique condensé portant au moins deux groupes hydroxyle sur deux atomes de carbone consécutifs du cycle.

**11.** Utilisation selon la revendication 10, **caractérisée en ce que** l'orthodiphénol est un composé de formule :

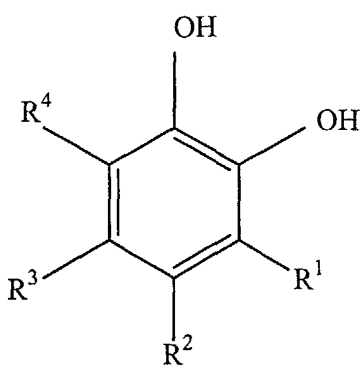

(I)

dans laquelle les substituts $R^1$ à $R^4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvait être éventuellement substitué, aryle, aryle substitué, un radical hétérocyclique éventuellement substitué, un radical contenant éventuellement un ou plusieurs atomes de silicium, où deux des substituants $R^1$ à $R^4$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

**12.** Utilisation selon la revendication 10, **caractérisée en ce que** le colorant d'oxydation est choisi parmi les flavanols,

les flavonols, les anthocyaninidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, et les mélanges de deux ou plus des composés précédents.

**13.** Utilisation selon la revendication 12, **caractérisée en ce que** le colorant d'oxydation est le 5,6-dihydroxyindole ou l'acide 5,6-dihydroxyindole carboxylique.

**14.** Utilisation selon l'une quelconque des revendications 10 à 13, **caractérisée en ce que** le colorant orthodiphénol est contenu dans des extraits de plantes, de fruits, d'agrumes, de légumes ou leurs mélanges.

**15.** Utilisation selon la revendication 14, **caractérisée en ce que** le colorant d'oxydation orthodiphénol est contenu dans des extraits de thé, de raisin, de pomme, de banane, de pomme de terre ou leurs mélanges.

**16.** Utilisation selon l'une quelconque des revendications précédentes, **caractérisée en ce que** la composition pour la coloration des fibres kératiniques comprend en outre un second colorant d'oxydation choisi parmi les bases d'oxydation, les coupleurs et leurs mélanges.

**17.** Utilisation selon la revendication 16, **caractérisée en ce que** les bases d'oxydation sont choisies parmi les ortho- et para-phénylènediamines, les bases doubles, les ortho- et para-aminophénols, les bases hétérocycliques, ainsi que les sels d'addition de tous ces composés avec un acide.

**18.** Utilisation selon la revendication 17, **caractérisée en ce que** la base d'oxydation est choisie parmi les para-phénylènediamines répondant à la formule (II) :

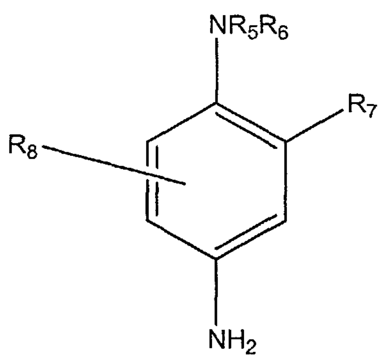

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;
- $R_8$ représente un atome d'hydrogène, d'halogène ou un radical alkyle en $C_1$-$C_4$ ;
- $R_5$ et $R_6$ peuvent également former avec l'atome d'azote qui les porte un hétérocycle azoté à 5 ou 6 chaînons éventuellement substitué par un ou plusieurs groupements alkyle, hydroxy ou uréido.

**19.** Utilisation selon la revendication 18, **caractérisée en ce que** les para-phénylènes diamines sont choisies parmi

la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

**20.** Utilisation selon la revendication 17, **caractérisée en ce que** les bases doubles sont choisies parmi les composés répondant à la formule (III) :

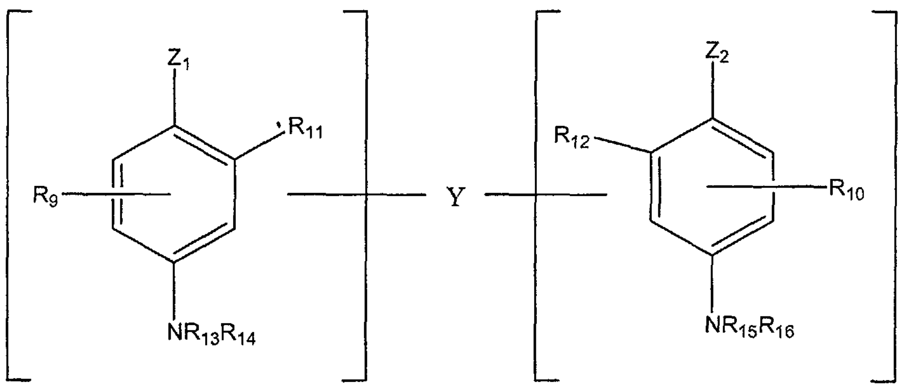

(III)

dans laquelle :

- $Z_1$ et $Z_2$, identiques ou différents, représentent un radical hydroxyle ou -NH2 pouvant être substitué par un radical alkyle en $C_1$-$C_4$ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ; étant entendu que les composés de formule (III) ne comportent qu'un seul bras de liaison Y par molécule.

**21.** Utilisation selon la revendication 20, **caractérisée en ce que** les bases doubles sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

**22.** Utilisation selon la revendication 17, **caractérisée en ce que** la base d'oxydation est choisie parmi les para-aminophénols répondant à la formule (IV)

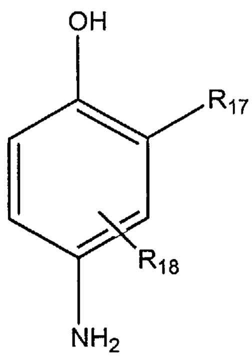

(IV)

dans laquelle :

- $R_{17}$ représente un atome d'hydrogène,un atome d'halogène tel que le fluor ou le chlore, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl ($C_1$-$C_4$)aminoalkyle en $C_1$-$C_4$ ;
- $R_{18}$ représente un atome d'hydrogène ou un atome d'halogène tel que le fluor ou le chlore, un radical alkyle en $C_1$-$C_4$, monohydroxyalkyle en $C_1$-$C_4$, polyhydroxyalkyle en C2-C4, aminoalkyle en $C_1$-$C_4$, cyanoalkyle en $C_1$-$C_4$ ou alcoxy($C_1$-$C_4$)alkyle($C_1$-$C_4$).

**23.** Utilisation selon la revendication 22, **caractérisée en ce que** les para-aminophénols sont choisis parmi le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

**24.** Utilisation selon la revendication 17, **caractérisée en ce que** la base d'oxydation est un orthoaminophénol choisi parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

**25.** Utilisation selon la revendication 17, **caractérisée en ce que** la base d'oxydation est une base hétérocyclique choisie parmi les dérivés pyridiniques, les dérivés pyrimidiniques, les dérivés pyrazoliques, et leurs sels d'addition avec un acide.

**26.** Utilisation selon la revendication 25, **caractérisée en ce que** les dérivés pyridiniques sont choisis parmi la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, et la 3,4-diamino-pyridine.

**27.** Utilisation selon la revendication 25, **caractérisée en ce que** les dérivés pyrimidiniques sont choisis parmi la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique.

**28.** Utilisation selon la revendication 25, **caractérisée en ce que** les dérivés pyrazoliques sont choisis parmi le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, et le 3,5-diamino-4-(β-hydroxyéthyl)amino-1-méthyl-pyrazole.

**29.** Utilisation selon l'une quelconque des revendications précédentes, **caractérisée en ce que** la base d'oxydation représente de 0,0005 à 12% en poids du poids total de la composition, et de préférence de 0,005 à 8%.

**30.** Utilisation selon la revendication 16, **caractérisée en ce que** les coupleurs sont choisis parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

**31.** Utilisation selon la revendication 30, **caractérisée en ce que** les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

**32.** Utilisation selon la revendication 30 ou 31, **caractérisée en ce que** le ou les coupleurs représentent de 0,0001 à 15% en poids du poids total de la composition, et de préférence de 0,001 à 10%.

**33.** Utilisation selon l'une quelconque des revendications précédentes, **caractérisée en ce qu'**elle comprend un ou plusieurs acides aminés et/ou une ou plusieurs protéines.

**34.** Utilisation selon la revendication 38, **caractérisée en ce que** le ou les acides aminés comprennent au moins un groupe thiol et sont choisis parmi les acides aminés ayant une fonction amine en position α par rapport à une fonction acide carboxylique.

**35.** Utilisation selon la revendication 34, **caractérisée en ce que** le ou les acides aminés sont choisis parmi la cystéine et ses dérivés, les protéines sont choisies parmi le glutathion et ses dérivés.

**36.** Utilisation selon l'une quelconque des revendications 33 à 35, **caractérisée en ce que** le rapport molaire du (ou des) acide(s) aminé(s) et du (ou des) protéines au(x) colorant(s) d'oxydation varie de 0,001 à 50, de préférence de 0,01 à 5, et mieux de 0,05 à 2,5.

**37.** Utilisation selon l'une quelconque des revendications précédentes, **caractérisée en ce que** la composition comprend en outre au moins une enzyme.

**38.** Utilisation selon la revendication 37, **caractérisée en ce que** l'enzyme est choisie parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases, les superoxydesdimutases et leurs mélanges, ou parmi des extraits végétaux ou animaux contenant des enzymes précitées.

**39.** Utilisation selon la revendication 38, **caractérisée en ce que** l'enzyme est choisie parmi les tyrosinases.

**40.** Utilisation selon la revendication 37 à 39, **caractérisée en ce qu'**elle comprend $5.10^{-3}$ à 5 mg, de préférence $5.10^{-2}$ à 0.5 mg d'enzyme par millilitre de composition finale.

**41.** Utilisation selon l'une quelconque des revendications précédentes, **caractérisée en ce que** le colorant d'oxydation

orthodiphénol est présent en une quantité de 1 mM à 10 mM par litre de composition.

**42.** Utilisation selon l'une quelconque des revendications précédentes, **caractérisée en ce que** le milieu physiologiquement acceptable est un milieu solubilisant du colorant d'oxydation, de préférence à propriété bactériologique.

**43.** Utilisation selon l'une quelconque des revendications précédentes, **caractérisée en ce que** le milieu physiologiquement acceptable comprend un solvant ou un mélange de solvants du colorant.

**44.** Utilisation selon la revendication 43, **caractérisée en ce que** le solvant est choisi parmi l'eau, les alcools, les éthers, le diméthylsulfoxyde, la N-méthylpyrrolidone, les acétones et leurs mélanges.

**45.** Utilisation selon la revendication 44, **caractérisée en ce que** l'alcool est un alcanol ou un alcanediol.

**46.** Utilisation selon la revendication 44, **caractérisée en ce que** le solvant est un mélange eau/alcool.

**47.** Utilisation selon la revendication 46, **caractérisée en ce que** l'alcool représente jusqu'à 80% en poids du mélange, de préférence 1 à 50% en poids et mieux de 5 à 20% en poids.

**48.** Utilisation selon l'une quelconque des revendications précédentes, **caractérisée en ce que** la composition est exempte de tout agent de chélation du sel de Mn(II) et/ou Zn(II).

**49.** Utilisation selon l'une quelconque des revendications précédentes, **caractérisée en ce que** la composition se présente sous la forme de deux composants séparés, un premier composant comprenant le système dissous dans un milieu physiologiquement acceptable et un deuxième composant comprenant le colorant d'oxydation dissous dans un milieu physiologiquement acceptable.

**50.** Utilisation selon l'une quelconque des revendications 1 à 48, **caractérisée en ce que** la composition est conditionnée sous forme d'un aérosol ou d'un système à pompe sans reprise d'air.

**51.** Utilisation selon la revendication 50, **caractérisée en ce que** la composition est conditionnée dans un système à pompe à deux compartiments distincts, chacun des composants étant contenu séparément dans un des deux compartiments.

**52.** Utilisation selon l'une quelconque des revendications 1 à 48, **caractérisée en ce que** la composition est conditionnée sous forme d'un galet.

**53.** Utilisation selon la revendication 52, **caractérisée en ce que** le galet comprend un excipient contenant de l'acide citrique et /ou tartrique en quantité sous stoechiométrique par rapport à l'hydrogénocarbonate alcalin et/ou alcalino terreux.

**54.** Utilisation selon l'une quelconque des revendication 1 à 48, **caractérisée en ce que** la composition est conditionnée sous forme de deux galets, un premier galet comprenant le système et un excipient et un second galet comprenant le colorant et un excipient.

**55.** Utilisation selon l'une quelconque des revendications 52 à 54, **caractérisée en ce que** le ou les galets sont des galets effervescents.

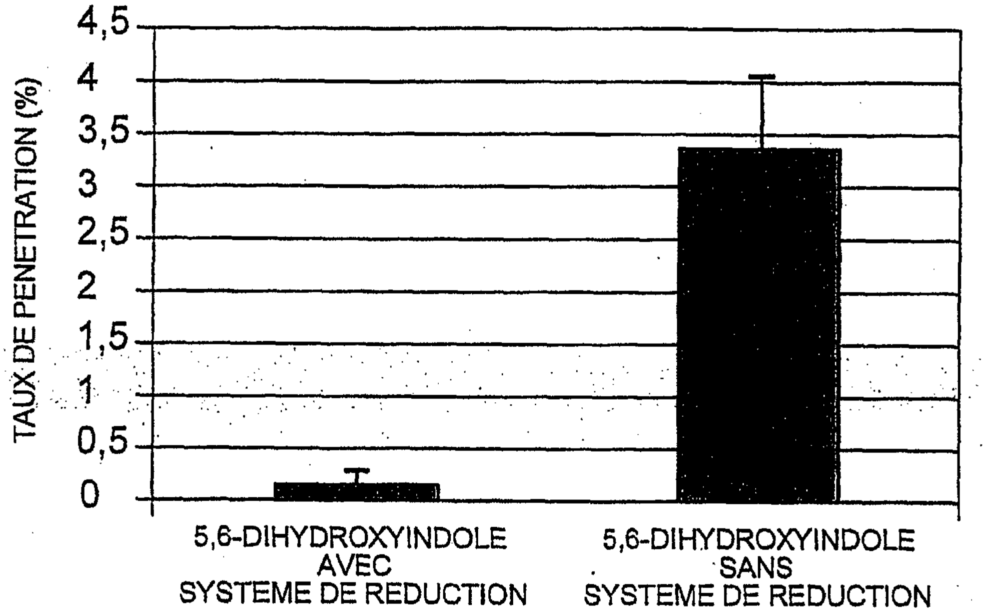

TAUX DE PENETRATION (%)
4,5
4
3,5
3
2,5
2
1,5
1
0,5
0
5,6-DIHYDROXYINDOLE AVEC SYSTEME DE REDUCTION
5,6-DIHYDROXYINDOLE SANS SYSTEME DE REDUCTION


FIGURE 1